# EUROPEAN PATENT APPLICATION

(11) **EP 3 222 540 A1**
(43) Date of publication of application: **27.09.2017**
(21) Application number: 15861399.2
(22) Date of filing: 17.11.2015
(51) Int. Cl.: B65B 55/08, A61L 2/08, B65B 55/04

(54) **ELECTRON BEAM STERILIZATION DEVICE**

(30) Priority: 18.11.2014 JP 2014233188
(71) Applicant: Hitachi Zosen Corporation, Osaka-shi, Osaka 559-8559 (JP)
(72) Inventor: SASAKI Kazuaki, Osaka-shi, Osaka 559-8559 (JP); YOKOBAYASHI Takayasu, Osaka-shi, Osaka 559-8559 (JP); TAKEDA Shinichi, Osaka-shi, Osaka 559-8559 (JP)
(74) Representative: Engelhardt, Harald
(86) International application number: PCT/JP2015/082177
(87) International publication number: WO 2016/080365

(57) **Abstract**

An electron beam sterilization device (1) includes a rotational member (22) that rotates around vertical rotational shafts (33), (34), and the rotational member (22) includes an upper stage (25), a middle stage (27), and a lower stage (26). On the upper stage (25), there are provided a plurality of electron beam emission units (15) arranged along the circumferential direction. On the middle stage (27), there is provided a retaining device (17) that retains a container (2). On the lower stage (26), there are provided a plurality of power supply units (71) that supply power to the electron beam emission units (15). Power supply cables (72) extend from the power supply units (71) to the upper stage (25) along the rotational shafts (33), (34) and are connected to the electron beam emission units (15).

## Description

### TECHNICAL FIELD

The present invention relates to an electron beam sterilization device for applying an electron beam onto an inner surface of containers for foods, liquid drugs or the like being conveyed, thereby to sterilize the containers.

### BACKGROUND

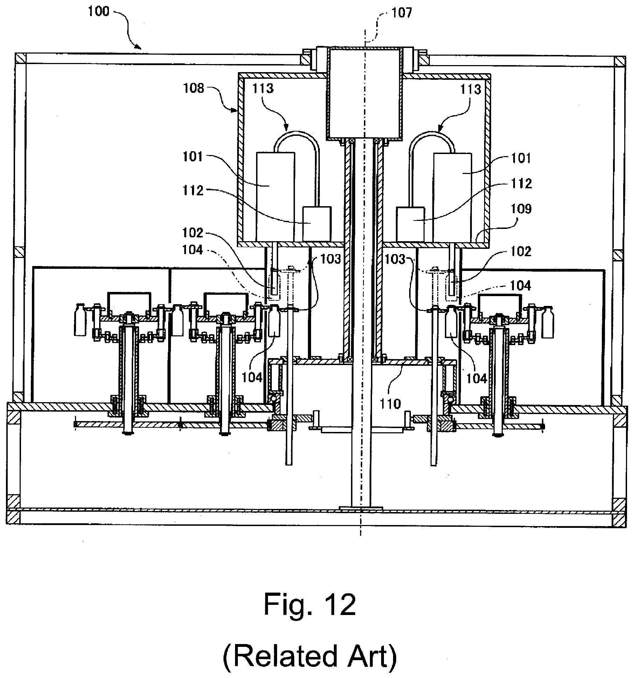

Such electron beam sterilization devices have conventionally been configured as shown in Fig. 12, in which a plurality of electron beam emission units 101 have nozzles 102, and containers 104 retained by a plurality of retaining devices 103 are moved vertically to insert the nozzles 102 into the containers 104, and electron beams are emitted from emission windows of the nozzles 102 to sterilize the interiors of the containers 104.

Such electron beam sterilization devices 100 include a rotational member 108 to be rotationally driven around a vertical rotational axis 107 by a rotational drive unit. The rotational member 108 includes upper and lower rotational tables 109, 110 having a disk shape.

The electron beam emission units 101 are arranged on the outer peripheral portion of the upper rotational table 109 at regular angular intervals in the circumferential direction. Each of the electron beam emission units 101 includes a nozzle 102 projecting downward.

The retaining devices 103 are provided on the lower rotational table 110 of the rotational member 108 so as to be capable of elevation and are opposed to the associated nozzles 102 from below. On the upper rotational table 109, there may be provided a plurality of power supply units 112 for supplying power to the electron beam emission units 101. The power supply units 112 and the electron beam emission units 101 are electrically connected to each other via power supply cables 113.

With this arrangement, the rotational member 108 rotates while the containers 104 are retained by the retaining devices 103, and as shown by the virtual line in Fig. 12, the retaining devices 103 are elevated so as to elevate the containers 104 and insert the nozzles 102 into the containers 104. Electron beams 105 are emitted from the emission windows of the nozzles 102 to sterilize the interiors of the containers 104.

The electron beam sterilization devices 100 including the rotational member 108 as described above are disclosed in, for example, Patent Literature 1.

### RELEVANT REFERENCES

### LIST OF RELEVANT PATENT LITERATURE

Patent Literature 1: Japanese Patent Application Publication No. 2013-86822

### SUMMARY

In the above-described conventional arrangement, the power supply units 112 are provided on the upper rotational table 109, and thus are positioned in an upper portion of the rotational member 108. Since the power supply units 112 have a large weight, the upper portion of the rotational member 108 has a large weight due to the weight of the power supply units 112. The center of gravity of the whole rotational member 108 is positioned high, and therefore, the rotational member 108 tends to rotate unstably.

One object of the present invention is to provide an electron beam sterilization device in which the rotational member rotates stably.

To achieve the above object, the first aspect of the present invention pertains to an electron beam sterilization device in which at least one of nozzles of electron beam emission units and containers are moved relative to the other so as to insert the nozzles into the containers through opening portions thereof, and interiors of the containers are sterilized by electron beams emitted from emission windows of the nozzles, the electron beam sterilization device comprising: an upper stage, a middle stage, and a lower stage provided in a rotational member configured to be rotated around a vertical rotational shaft; a plurality of electron beam emission units arranged on the upper stage along a circumferential direction and having the nozzles projected downward; a plurality of retaining devices arranged on the middle stage in association with the nozzles and configured to retain the containers; a plurality of power supply units arranged on the lower stage and configured to supply power to the electron beam emission units, respectively; and a plurality of power supply cables extending from the power supply units to the upper stage along the rotational shaft and connected to the plurality of electron beam emission units, respectively.

In this arrangement, the power supply units are provided on the lower stage of the rotational member, and therefore, the center of gravity of the whole rotational member is lowered, thereby stabilizing the rotation of the rotational member. In the electron beam sterilization device according to the second aspect of the present invention, the electron beam emission units and the power supply units are arranged in phase, and the electron beam emission units and the power supply units connected via same power supply cables are arranged in phases differentiated in a circumferential direction of the rotational member, whereby at least portions of the power supply cables extending from the power supply units toward the rotational shaft in the lower stage have a large radius of curvature.

In this arrangement, the electron beam emission units and the power supply units connected via same power supply cables are arranged at positions differentiated in the circumferential direction of the rotational member. Therefore, the power supply cables extending from the power supply units can be bent along the circumferential direction of the rotational member toward the rotational shaft, whereby the power supply cables can have a large radius of curvature in the lower stage. This enables a thick power supply cable to be bent easily and facilitates wiring of the power supply cable.

In the electron beam sterilization device according to the third aspect of the present invention, the middle stage of the rotational member has a plurality of elevation units for elevating and lowering the containers so as to insert the nozzles into the opening portions. In this arrangement, the containers are elevated and lowered by the elevation units so as to insert the nozzles of the electron beam emission units into the containers, and electron beams are emitted from the emission windows of the nozzles to sterilize the interiors of the containers.

In the electron beam sterilization device according to the fourth aspect of the present invention, the rotational member includes a blocking cylinder, the power cables extend from the power supply units through the blocking cylinder and are connected to the electron beam emission units, and the blocking cylinder is made of a blocking member configured to block at least one of a magnetic field generated from the power supply cables and radiations generated from the electron beam emission units.

In this arrangement, the blocking cylinder blocks at least one of a magnetic field generated from the power supply cables and radiations generated from the electron beam emission units. Therefore, when the magnetic field is blocked, the electron beams emitted from the nozzles of the electron beam emission units can be prevented from being bent by the magnetic field generated from the power supply cables, and it can be ensured that the electron beams are applied to the interiors of the containers and the interiors of the containers are sterilized.

When the radiations are blocked, the power supply cables can be prevented from being deteriorated due to the radiations.

### ADVANTAGES

With the present invention as described above, the rotational member rotates stably, ensuring that the electron beams are emitted into the containers and the interiors of the containers are sterilized.

### BRIEF DESCRIPTION OF THE DRAWINGS

Fig. 1 is a longitudinal sectional view of an electron beam sterilization device according to an embodiment of the present invention.
Fig. 2 shows a plastic bottle to be sterilized by the electron beam sterilization device of the embodiment.
Fig. 3 is a schematic plan view of a plurality of chambers arranged in the electron beam sterilization device of the embodiment.
Fig. 4 is a sectional view of a clamp unit, an elevation unit, and an opening/closing mechanism of the electron beam sterilization device of the embodiment.
Fig. 5 is a plan view showing an arrangement of electron beam emission units, power supply units, and power supply cables of the electron beam sterilization device of the embodiment.
Fig. 6 is a plan view showing a positional relationship between an electron beam emission unit and a power supply unit connected to a same power supply cable of the electron beam sterilization device of the embodiment.
Fig. 7 is a schematic perspective view showing a positional relationship between an electron beam emission unit and a power supply unit connected to a same power supply cable of the electron beam sterilization device of the embodiment.
Fig. 8 is a view along the line X-X in Fig. 1.
Fig. 9 is a plan view of the clamp unit of the electron beam sterilization device of the embodiment.
Fig. 10 is an enlarged cross-sectional view of a part of Fig. 8.
Fig. 11 shows a preform product to be sterilized by the electron beam sterilization device of the embodiment.
Fig. 12 is a longitudinal sectional view of a conventional electron beam sterilization device.

### DESCRIPTION OF EXAMPLE EMBODIMENTS

Embodiments of the present invention will be hereunder described with reference to the accompanying drawings.

### First Embodiment

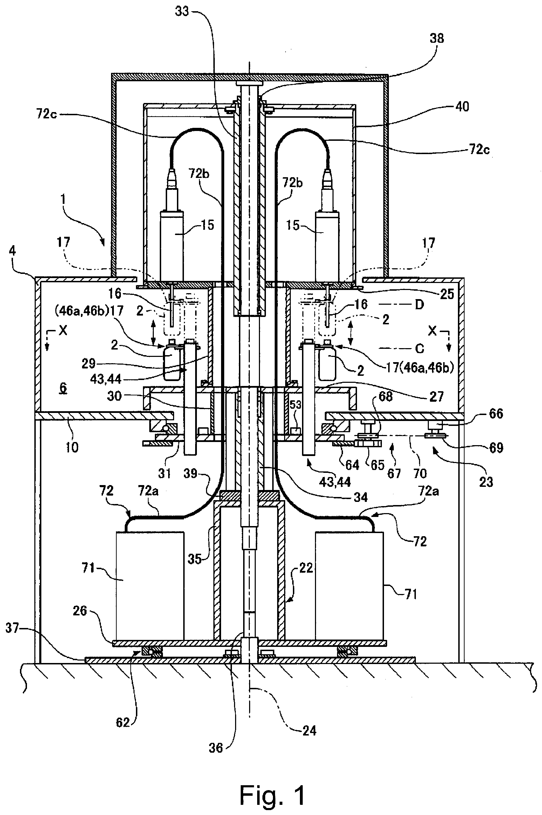

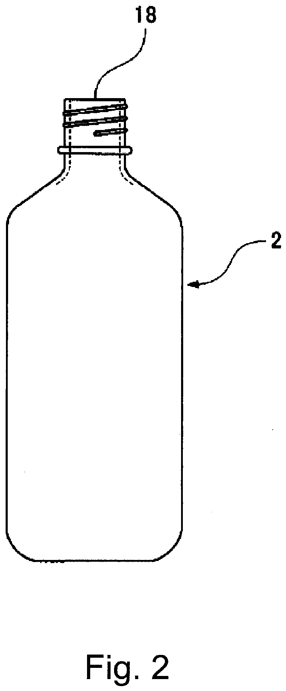

In the first embodiment, an electron beam sterilization device 1 may apply electron beams to plastic bottles 2 (an example of bottles) for sterilization, as shown in Figs. 1 and 2. The plastic bottles 2 may be formed from, for example, a preform product having a test tube shape (with a U-shaped longitudinal section opened at the upper end thereof) subjected to blow molding.

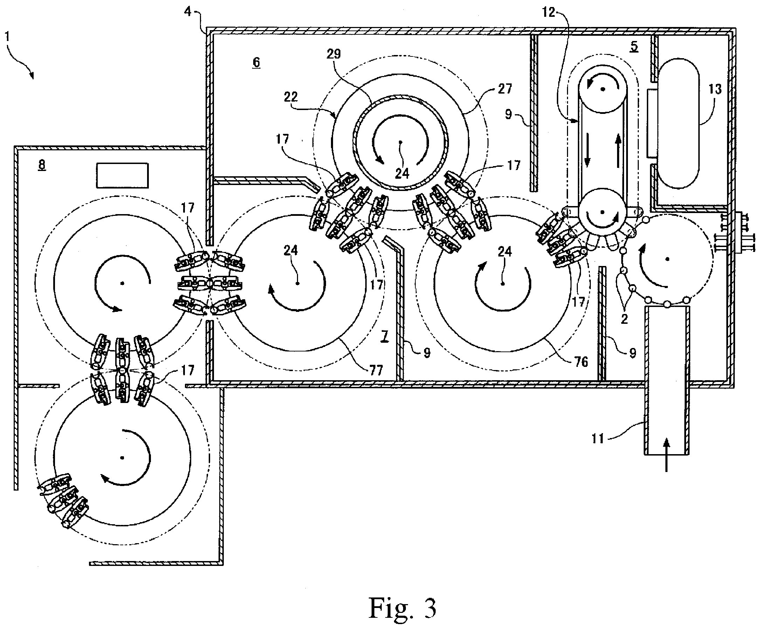

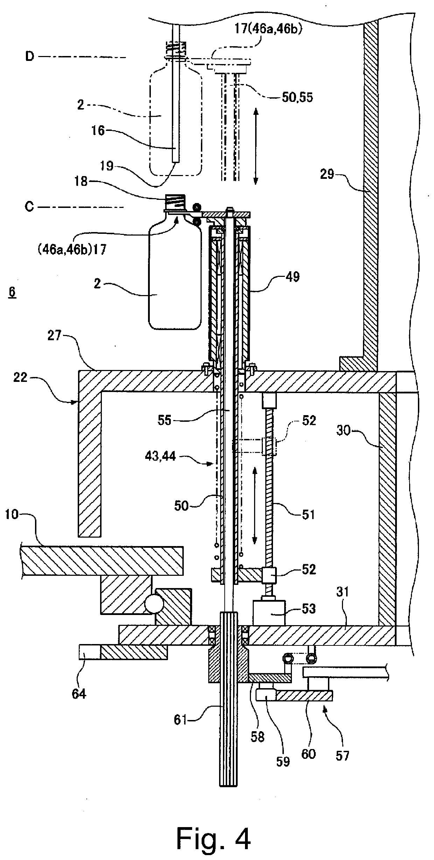

As shown in Fig. 3, the electron beam sterilization device 1 may generally include four chambers 5 to 8 in a cover member 4. The chambers 5 to 8, which may be arranged from the upstream side of the conveyance path of the plastic bottles 2, may include an outer surface sterilization chamber 5 in which electron beams are applied to outer surfaces of the plastic bottles 2 fed from outside so as to perform sterilization, an inner surface sterilization chamber 6 in which electron beams are applied to inner surfaces of the plastic bottles 2 so as to perform sterilization, a blocking chamber 7 for blocking radiations produced by application of the electron beams to the plastic bottles 2, and a sorting chamber 8 in which the plastic bottles 2 that are insufficiently sterilized (hereinafter referred to simply as "defective products") are ejected. In the cover member 4, there may be provided a plurality of partition walls 9 for defining the chambers 5 to 8 and an intermediate frame 10 (see Figs. 1 and 4) positioned horizontally.

The outer surface sterilization chamber 5 may include an input portion 11 through which plastic bottles 2 are put in from outside, a conveyor portion 12 for conveying the plastic bottles 2 having been put in, and an outer-surface electron beam emission unit 13 for applying electron beams to outer surfaces of the plastic bottles 2 being conveyed by the conveyor portion 12.

As shown in Figs. 1 and 4, in the inner surface sterilization chamber 6, the plastic bottles 2 retained by the clamp units 17 (an example of the retaining devices) may be moved vertically with respect to the nozzles 16 of the electron beam emission units 15, so as to insert the nozzles 16 into the plastic bottles 2 through the opening portions 18, and electron beams may be emitted from emission windows 19 of the nozzles 16 to sterilize the interiors of the plastic bottles 2.

The inner surface sterilization chamber 6 may include the rotational member 22 that may be rotationally driven around a vertical rotational axis 24 by the rotational drive unit 23. The rotational member 22 may include an upper rotational table 25 (an example of the upper stage), a lower rotational table 26 (an example of the lower stage), an intermediate rotational table 27 (an example of the middle stage) provided between the upper rotational table 25 and the lower rotational table 26, a blocking cylinder 29 having a circular shape and provided between the upper rotational table 25 and the intermediate rotational table 27, a mounting cylinder 30 having a circular shape and provided on the lower surface of the intermediate rotational table 27, an intermediate mounting table 31 (an example of the middle stage) having a ring shape and provided on the lower end of the mounting cylinder 30, first and second rotational shafts 33, 34 having a cylindrical shape, a lower cylindrical frame 35, and a connection unit 39.

The first and second rotational shafts 33, 34 may be provided in the central portion of the rotational member 22 at an upper and lower positions, respectively. A fixed shaft 36 may extend through the first and second rotational shafts 33, 34 and may be mounted to a bottom frame 37 of the inner surface sterilization chamber 6. Both the first and second rotational shafts 33, 34 may be rotatably supported by the fixed shaft 36 via a bearing 38.

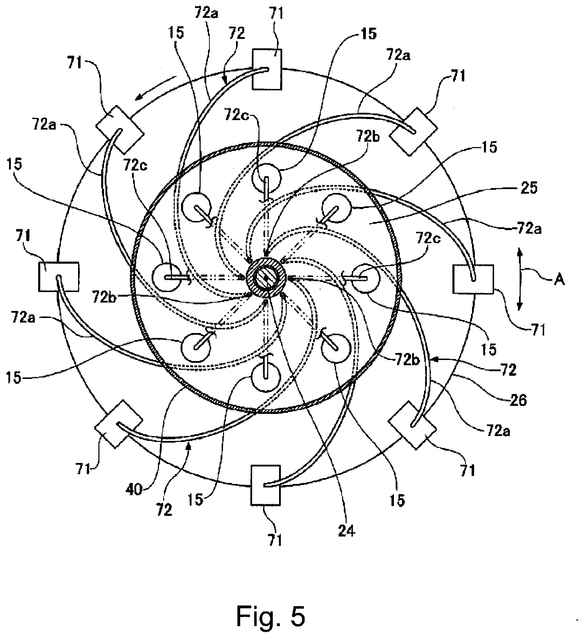

As shown in Figs. 1 and 5, the upper rotational table 25 may have a disk shape. A plurality of electron beam emission units 15, each projecting the nozzle 16 downward, may be arranged on the outer peripheral portion of the upper rotational table 25 at regular angular intervals. An upper blocking member 40 may be provided on the upper rotational table 25, and the electron beam emission units 15 may be positioned inside the upper blocking member 40.

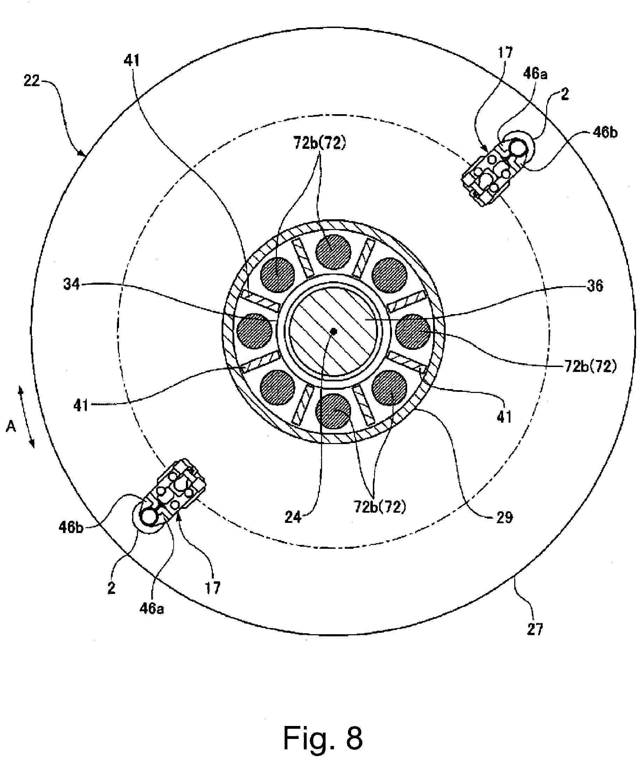

The intermediate rotational table 27 may have a disk shape. As shown in Fig. 8, there may be provided a plurality of partition plates 41 inside the blocking cylinder 29 at regular intervals in the circumferential direction A. As shown in Figs. 1 and 3, the clamp units 17 may be provided on the outer peripheral portion of the intermediate rotational table 27 at regular intervals. Further, on the outer peripheral portion of the intermediate rotational table 27, there may be provided a plurality of elevation units 43 for elevating and lowering the clamp units 17, and opening/closing mechanisms 44.

As shown in Figs. 1, 8, and 9, the clamp units 17 may be provided in association with the nozzles 16 and may each include a pair of clamp arms 46a, 46b capable of opening and closing and a spring 47 (an example of biasing member) that biases the pair of clamp arms 46a, 46b toward the respective closing directions.

As shown in Fig. 4, the elevation unit 43 may include a shaft sleeve 50 guided by a guide member 49 so as to be capable of elevation, a screw shaft 51 arranged in parallel with the shaft sleeve 50, an internal thread member 52 provided on a lower portion of the shaft sleeve 50 and threadably engaged with the screw shaft 51, and a servomotor 53 for rotationally driving the screw shaft 51. As shown in Fig. 1, the servomotor 53 may be mounted on the intermediate mounting table 31 of the rotational member 22.

The clamp arms 46a, 46b may be provided on the upper end of the shaft sleeve 50. When the servomotor 53 rotates the screw shaft 51, the internal thread member 52 may be moved in the axial direction of the screw shaft 51, and the clamp arms 46a, 46b may be elevated or lowered along with the shaft sleeve 50.

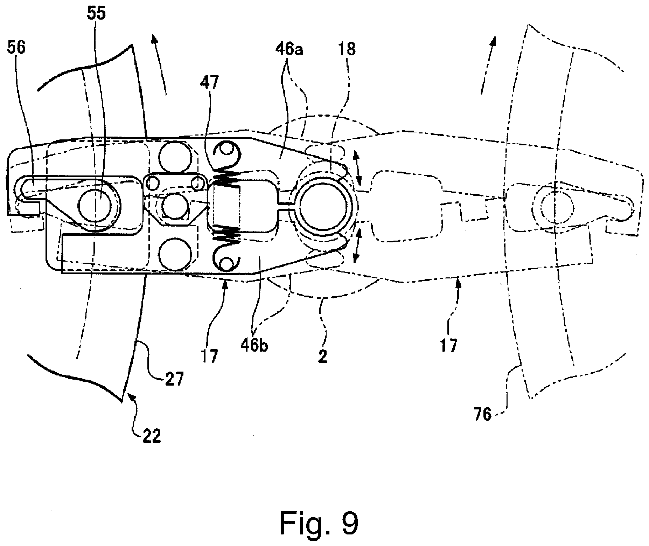

As shown in Figs. 4 and 9, the opening/closing mechanism 44 may operate to open and close the clamp arms 46a, 46b. The opening/closing mechanism 44 may include an inner shaft 55 inserted in the shaft sleeve 50 and capable of rotating around the axis thereof, a cam 56 provided on the upper end of the inner shaft 55, and a cam unit 57 for rotating the inner shaft 55. The cam unit 57 may include a cam lever 58 and a cam roller 59. The cam lever 58 may be fitted on a lower portion of the inner shaft 55 via a spline 61. The cam roller 59 may be provided on a distal end portion of the cam lever 58. The cam lever 58 may be mounted to the intermediate mounting table 31 so as to be capable of swinging.

The inner shaft 55 can be elevated and lowered along with the shaft sleeve 50 with respect to the cam lever 58. Further, the inner surface sterilization chamber 6 may include a fixed cam member 60. When the rotational member 22 rotates, the cam roller 59 may contact with the fixed cam member 60 to cause the cam lever 58 to swing. As a result, the inner shaft 55 may rotate and the cam 56 may swing, causing the clamp arms 46a, 46b to open or close.

As shown in Fig. 1 and Figs. 5 to 7, the lower rotational table 26 may have a disk shape and may be rotatably supported on the bottom frame 37 via a support member 62. The lower cylindrical frame 35 may be mounted on the lower rotational table 26. The lower end of the second rotational shaft 34 and the upper end of the lower cylindrical frame 35 may be connected via the connection unit 39. The connection unit 39 may absorb the misalignment of the lower rotational table 26.

On the lower rotational table 26, there may be provided a plurality of power supply units 71 for supplying power to the electron beam emission units 15. The electron beam emission units 15 and the power supply units 71 may be associated with each other in one-to-one relationship and may be opposed to each other in the vertical direction. Since the power supply units 71 have a large weight, the rotational accuracy of the lower rotational table 26 may be maintained to be low, whereas the rotational accuracy of the intermediate rotational table 27 may be maintained to be high. The difference in the rotational accuracy may enable the connection unit 39 to absorb the misalignment occurring to the lower rotational table 26. As a result, the rotation of the rotational member 22 may not be hindered.

The electron beam emission units 15 and the power supply units 71 may be connected via the power supply cables 72. Each of the power supply cables 72 may include a lower wire portion 72a, a vertical wire portion 72b, and an upper wire portion 72c. The lower wire portion 72a may extend from the power supply unit 71 toward the rotational axis 24 and reach the second rotational shaft 34. The vertical wire portion 72b may extend upward from the lower wire portion 72a along the first and second rotational shafts 33, 34. The upper wire portion 72c may have a semicircular shape and may be curved from the upper end of the vertical wire portion 72b toward the outer periphery and connected to the electron beam emission unit 15.

For easier understanding, Fig. 5 shows eight electron beam emission units 15 and eight power supply units 71. However, this configuration is not limitative, and actually a larger number of electron beam emission units 15 and power supply units 71 may be provided.

As shown in Figs. 1 and 8, the vertical wire portion 72b of the power supply cable 72 may extend through the blocking cylinder 29 and the mounting cylinder 30. In the blocking cylinder 29, the partition wall 41 may be interposed between one vertical wire portion 72b and another adjacent thereto.

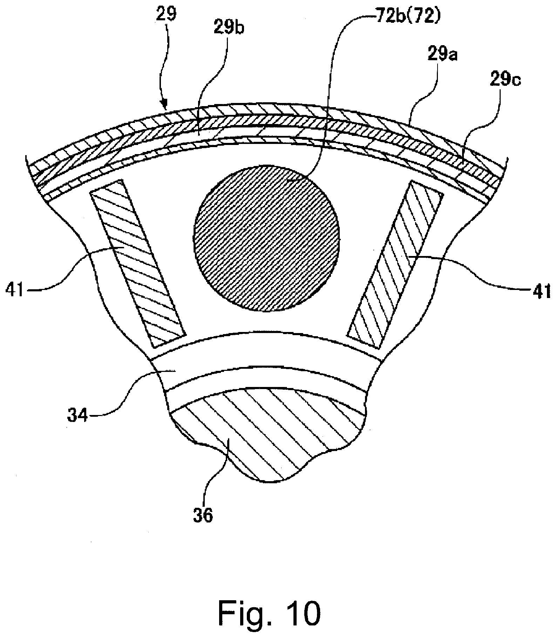

As shown in Fig. 10, the blocking cylinder 29 may include an outer member 29a having a high corrosion resistance and a magnetic field blocking member 29b and a radiation blocking member 29c provided inside the outer member 29a. The outer member 29a may be made of, e.g., stainless steel. The magnetic field blocking member 29b may be made of a material that can well block the magnetic field generated from the power supply cable 72, e.g., Mu-Metal having a high magnetic permeability (e.g., a permalloy having Mo, Mn, Cu, or Cr added thereto or 78Ni-5Mo-4Cu-Fe and 36Ni-Fe) or a high magnetic permeability metal (e.g., an alloy of iron and nickel). The radiation blocking member 29c may be made of a material that can well block the radiations generated from the electron beam emission unit 15. One example of such material is lead.

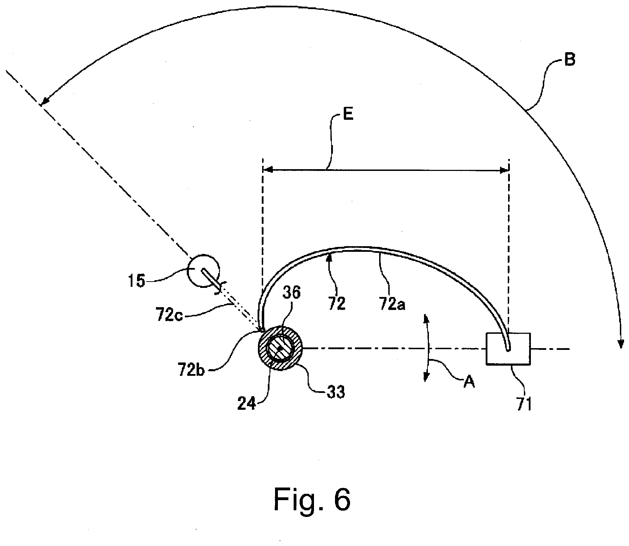

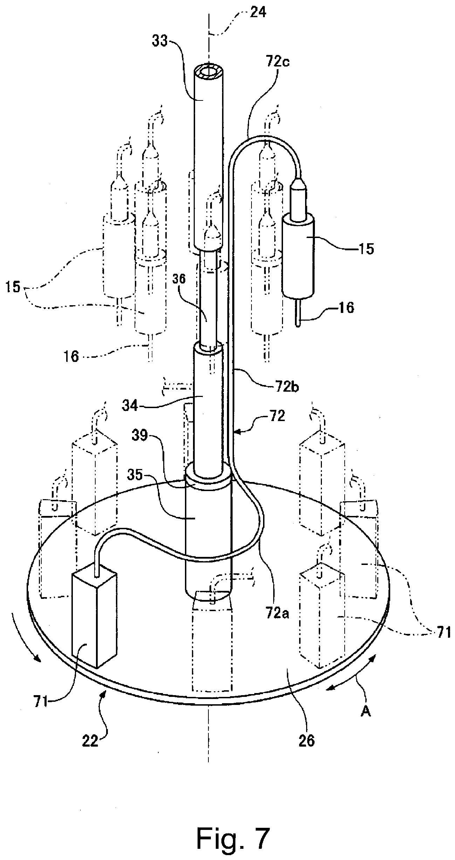

The electron beam emission units 15 and the power supply units 71 may be arranged in phase. More specifically, as shown in Fig. 5, the electron beam emission units 15 and the power supply units 71 provided therebelow may be arranged in the same positions in the circumferential direction A of the rotational member 22. However, the electron beam emission unit 15 and the power supply unit 71 connected via the same power supply cable 72 may be arranged in phases differentiated in the circumferential direction A of the rotational member 22. More specifically, as shown in Figs. 6 and 7, the electron beam emission unit 15 and the power supply unit 71 connected via the same power supply cable 72 may be arranged at positions differentiated by an angle B in the circumferential direction A of the rotational member 22.

Thus, the distance E from the power supply unit 71 to the vertical wire portion 72b of the power supply cable 72 may be larger. The lower wire portion 72a may be bent in an arc shape in the circumferential direction A of the rotational member 22 in a plan view, and may also be bent upward and continuous to the vertical wire portion 72b, as shown in Figs. 1 and 7.

As shown in Figs. 1 and 4, a ring gear 64 may be provided in the outer periphery of the intermediate mounting table 31 over the whole circumference thereof. The rotational drive unit 23 may include a drive gear 65 meshing with the ring gear 64, a motor 66, a transmission mechanism 67 that transmits the rotation of the motor 66 to the drive gear 65. The transmission mechanism 67 may include a pair of sprockets 68, 69 and a chain 70 wound around the sprockets 68, 69. The drive gear 65 and the motor 66 may be provided on the intermediate frame 10.

As shown in Fig. 3, the inner surface sterilization chamber 6 may include a turn rotation table 76 for receiving plastic bottles 2 conveyed from the outer surface sterilization chamber 5 and conveying them to the clamp units 17 of the rotational member 22. Further, the blocking chamber 7 may include a sterilized rotation table 77 for receiving the plastic bottles 2 from the rotational member 22 of the inner surface sterilization chamber 6.

Both the turn rotation table 76 and the sterilized rotation table 77 may be rotationally driven around the respective vertical rotational axes 24, as with the rotational member 22. In addition, as with the rotational member 22, each of the turn rotation table 76 and the sterilized rotation table 77 may be provided with a plurality of clamp units 17 (see the virtual lines in Fig. 9). While the turn rotation table 76, the rotational member 22, and the sterilized rotation table 77 rotate, the plastic bottles 2 can be passed from the clamp units 17 of the turn rotation table 76 to the clamp units 17 of the rotational member 22, and the plastic bottles 2 can also be passed from the clamp units 17 of the rotational member 22 to the clamp units 17 of the sterilized rotation table 77.

The operations in the above arrangement will now be described. When the motor 66 drives, the rotation of the motor 66 may be transmitted to the ring gear 64 on the intermediate mounting table 31 via the transmission mechanism 67, and the rotational member 22 may be rotated. As shown in Fig. 3, while the turn rotation table 76 and the rotational member 22 in the inner surface sterilization chamber 6 and the sterilized rotation table 77 in the blocking chamber 7 rotate, and the plastic bottles 2 are put into the outer surface sterilization chamber 5 and conveyed, electron beams may be applied from the outer-surface electron beam emission unit 13 to the outer surfaces of the plastic bottles 2, thereby to sterilize the outer surfaces of the plastic bottles 2. As shown in Fig. 1, the rotation of the rotational member 22 may be integrated with the rotation of the clamp units 17, the elevation units 43, the opening/closing mechanisms 44, the electron beam emission units 15, the power supply units 71, and the power supply cables 72.

Then, the plastic bottles 2 may be conveyed from the outer surface sterilization chamber 5, retained by the clamp units 17 of the turn rotation table 76 of the inner surface sterilization chamber 6, and passed from the clamp units 17 of the turn rotation table 76 to the clamp units 17 of the rotational member 22. At this time, the clamp arms 46a, 46b of the turn rotation table 76 may switch from the closed state and open, while the clamp arms 46a, 46b of the rotational member 22 may switch from the opened state and close, whereby the plastic bottles 2 may be retained by the clamp units 17 of the rotational member 22.

As shown in Fig. 4, the servomotor 53 of the elevation unit 43 may operate to rotate the screw shaft 51, and the shaft sleeve 50 and the inner shaft 55 may be moved upward along with the internal thread member 52. As represented by the virtual lines in Fig. 4, the clamp arms 46a, 46b may be moved from the lowest position C to the highest position D. Thus, as represented by the virtual lines in Figs. 1 and 4, the plastic bottle 2 retained by the clamp arms 46a, 46b may be raised to the highest position D, such that the nozzle 16 of the electron beam emission unit 15 is inserted into the plastic bottle 2 through the opening portion 18 thereof. The interior of the plastic bottle 2 may be sterilized by the electron beam emitted from the emission window 19 of the nozzle 16.

After the sterilization is complete, the servomotor 53 of the elevation unit 43 may operate reversely to rotate the screw shaft 51 reversely, and the shaft sleeve 50 and the inner shaft 55 may be moved downward along with the internal thread member 52. As represented by the solid lines in Fig. 4, the clamp arms 46a, 46b may be moved from the highest position D to the lowest position C. Thus, as represented by the solid lines in Figs. 1 and 4, the plastic bottle 2 retained by the clamp arms 46a, 46b may be lowered to the lowest position C, such that the nozzle 16 of the electron beam emission unit 15 is separated upward from the plastic bottle 2.

Then, as shown in Fig. 3, the plastic bottles 2 may be passed from the clamp units 17 of the rotational member 22 to the clamp units 17 of the sterilized rotation table 77. At this time, the clamp arms 46a, 46b of the rotational member 22 may switch from the closed state and open, while the clamp arms 46a, 46b of the sterilized rotation table 77 may switch from the opened state and close, whereby the plastic bottles 2 may be retained by the clamp units 17 of the sterilized rotation table 77.

As shown in Figs. 1 and 7, the power supply unit 71 having a large weight may be provided on the lower rotational table 26 of the rotational member 22, and therefore, the center of gravity of the whole rotational member 22 may be lowered, thereby stabilizing the rotation of the rotational member 22.

Further, as shown in Figs. 1, 8, and 10, the vertical wire portions 72b of the power supply cables 72 may extend through the blocking cylinder 29 and the mounting cylinder 30, and therefore, the magnetic fields generated from the vertical wire portions 72b may be blocked by the magnetic field blocking member 29b of the blocking cylinder 29, and the electron beams emitted from the nozzles 16 of the electron beam emission units 15 can be prevented from being bent by the magnetic field generated from the power supply cable 72. Thus, it can be ensured that the electron beams are applied to the interiors of the plastic bottles 2 and the interiors of the plastic bottles 2 are sterilized.

The radiations generated from the electron beam emission units 15 may be blocked by the radiation blocking member 29c of the blocking cylinder 29, and therefore, the vertical wire portions 72b of the power supply cables 72 can be prevented from being deteriorated by the radiations.

Further, as shown in Figs. 6 and 7, the electron beam emission unit 15 and the power supply unit 71 connected via the same power supply cable 72 may be arranged at positions differentiated by an angle B in the circumferential direction A of the rotational member 22, and the lower wire portion 72a of the power supply cable 72 may be bent in an arc shape in the circumferential direction A, and also bent upward and continuous to the vertical wire portion 72b. Therefore, the portion of the power supply cable 72 connecting between the lower wire portion 72a and the vertical wire portion 72b can have a large radius of curvature. This may enable a thick power supply cable 72 to be bent easily and may facilitate wiring of the power supply cable 72.

In the above embodiment, as shown in Fig. 10, the blocking cylinder 29 may include the outer member 29a, the magnetic field blocking member 29b, and the radiation blocking member 29c. It may also be possible that the blocking cylinder 29 does not include the magnetic field blocking member 29b or the radiation blocking member 29c.

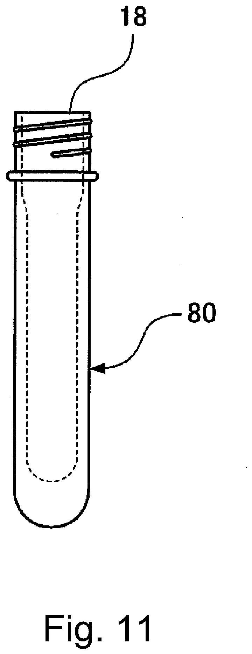

As shown in Fig. 2, the above embodiment is used for plastic bottles 2 formed from preform products subjected to blow molding, as an example of containers. However, the containers are not limited to the plastic bottles 2. For example, it may also be possible that, as shown in Fig. 11, the containers are preform products 80 having a test tube shape prior to blow molding, bags, or the like.

In the above embodiment, as shown in Fig. 1, the electron beam emission units 15 may be fixed in the vertical direction, and the plastic bottles are elevated and lowered by the elevation units 43, so as to insert the nozzles 16 of the electron beam emission units 15 into the plastic bottles 2 and pull it out. It may also be possible that the plastic bottles 2 are fixed in the vertical direction, and the electron beam emission units 2 are elevated and lowered by the elevation units 43, so as to insert the nozzles 16 of the electron beam emission units 15 into the plastic bottles 2 and pull it out. Further, it may also be possible that both the plastic bottles 2 and the electron beam emission units 2 are elevated and lowered by the elevation units 43, so as to insert the nozzles 16 of the electron beam emission units 15 into the plastic bottles 2 and pull it out.

In the above embodiment, as shown in Fig. 5, the lower wire portions 72a of the power supply cables 72 may be bent in an arc shape in the circumferential direction A of the rotational member 22. Likewise, it may also be possible that the upper wire portions 72c of the power supply cables 72 are bent in an arc shape in the circumferential direction A of the rotational member 22.

## Claims

1. An electron beam sterilization device in which at least one of containers and nozzles of electron beam emission units are moved relative to the other so as to insert the nozzles into the containers through opening portions thereof, and interiors of the containers are sterilized by electron beams emitted from emission windows of the nozzles, the electron beam sterilization device comprising:
an upper stage, a middle stage, and a lower stage provided in a rotational member configured to be rotated around a vertical rotational shaft;
a plurality of electron beam emission units arranged on the upper stage along a circumferential direction and having the nozzles projected downward;
a plurality of retaining devices arranged on the middle stage in association with the nozzles and configured to retain the containers;
a plurality of power supply units arranged on the lower stage and configured to supply power to the electron beam emission units, respectively; and
a plurality of power supply cables extending from the power supply units to the upper stage along the rotational shaft and connected to the plurality of electron beam emission units, respectively.

2. The electron beam sterilization device of claim 1, wherein
the electron beam emission units and the power supply units are arranged in phase, and
the electron beam emission units and the power supply units connected via same power supply cables are arranged in phases differentiated in a circumferential direction of the rotational member, whereby at least portions of the power supply cables extending from the power supply units toward the rotational shaft in the lower stage have a large radius of curvature.

3. The electron beam sterilization device of claim 1 or 2, wherein the middle stage of the rotational member has a plurality of elevation units for elevating and lowering the containers so as to insert the nozzles into the opening portions.

4. The electron beam sterilization device of claim 1 or 2, wherein
the rotational member includes a blocking cylinder,
the power cables extend from the power supply units through the blocking cylinder and are connected to the electron beam emission units, and
the blocking cylinder is made of a blocking member configured to block at least one of a magnetic field generated from the power supply cables and radiations generated from the electron beam emission units.
